**Europäisches Patentamt**

**European Patent Office**

**Office européen des brevets**

(19)

(11) Numéro de publication: **0 078 215**
**B1**

(12) **FASCICULE DE BREVET EUROPÉEN**

(45) Date de publication du fascicule du brevet:
**13.08.86**

(51) Int. Cl.⁴: **A 61 K 9/00,** A 61 J 3/06

(21) Numéro de dépôt: **82401957.4**

(22) Date de dépôt: **25.10.82**

(54) **Nouvelle forme galénique solide pour administration par voie orale et son procédé de préparation.**

(30) Priorité: **26.10.81 FR 8120048**

(43) Date de publication de la demande:
**04.05.83 Bulletin 83/18**

(45) Mention de la délivrance du brevet:
**13.08.86 Bulletin 86/33**

(84) Etats contractants désignés:
**AT BE CH DE FR GB IT LI LU NL SE**

(56) Documents cité:
**EP-A-0 072 469**
**FR-A-2 179 044**
**FR-A-2 343 473**
**US-A-3 439 089**

(73) Titulaire: **RHONE- POULENC SANTE, Les Miroirs
18 Avenue d'Alsace, F-92400 Courbevoie Cedex
(FR)**

(72) Inventeur: **Campan, Jean- Jacques, 12 rue Hudri,
F-92400 Courbeboie (FR)**
Inventeur: **Lombardi, Roberto, 63 avenue Foch,
F-78400 Chatou (FR)**

(74) Mandataire: **Pilard, Jacques, RHONE- POULENC
RECHERCHES Service Brevets Pharma 25, Quai
Paul Doumer, F-92408 Courbevoie Cedex (FR)**

## Description

La présente invention concerne une nouvelle forme galénique solide pour administration par voie orale et son procédé de préparation.

Les formes pharmaceutiques solides connues administrables par voie orale contenant une dose unitaire d'un ou plusieurs principes actifs sont principalement les comprimés, les dragées ou analogues et les gélules. Bien que l'industrie ait maîtrisé la préparation de telles formulations, il n'en reste pas moins que les procédés utilisés, en particulier pour la fabrication des comprimés sont longs et compliqués et ils impliquent des pertes pratiquement inévitables. Par exemples, le procédé de fabrication des comprimés est essentiellement un procédé discontinu qui nécessite le passage par, en général, 5 à 10 stades intermédiaires dont chacun doit être rigoureusement contrôle. De ce fait, l'ensemble du processus peut durer 1 à 2 semaines. A chaque stade intermédiaire, il y a inévitablement des pertes qu'il est très difficile de réduire. Il faut également noter que la multiplicité des stades de fabrication entraîne la multiplicité des installations de production ce qui a pour effet de rendre plus difficile le respect des pratiques de bonne fabrication (émission de poussières, contact du manipulateur avec le produit, .....).

Par ailleurs, la fabrication des comprimés se fait essentiellement par mélange de poudres de densité apparente et granulométrie différentes ce qui rend plus difficile l'obtention de doses vraiment uniformes du principe actif par comprimé et par suite l'obtention de lots de comprimés parfaitement homogènes ce qui présente un inconvénient majeur pour des principes actifs utilisés à très faibles doses.

Il est connu, d'après le brevet français FR 2 543 473 de préparer des comprimés dans lesquels la substance pharmaceutiquement active est mélangée à une substance porteuse de nature lipidique qui est solide à une température inférieure à 37°C et liquide à une température supérieure, de préférence, à 43°C.

Il a maintenant été trouvé, et c'est ce qui fait un des objets de la présente invention, une nouvelle forme galénique solide, appelée par la suite "forme orale solidifiée instantanée" ou "FOSI" qui présente sur les formes antérieurement connues l'avantage de pouvoir être préparée en continu plus rapidement et de conduire à des lots de doses unitaires dans lesquelles le principe actif est réparti très uniformément même dans le cas de faibles doses.

Selon l'invention, la "FOSI" est constituée d'un ou plusieurs principes actifs dissous ou dispersés dans un ou plusieurs excipients liquéfiables à une température supérieure à la température ambiante, par exemple 35°C, compatibles avec le ou les principes actifs et solides à la température ambiante et pouvant libérer le ou les principes actifs après dissolution dans l'eau ou un liquide physiologique.

Dans la forme galénique selon l'invention, le principe actif est dissous ou dispersé à l'état de particules beaucoup plus fines que dans les formes galéniques orales usuelles solides. Il en résulte principalement une modification de la biodisponibilité du principe actif qui se traduit au niveau de la libération et/ou de l'absorption du principe actif et par conséquent au niveau du métabolisme.

Les "FOSI" selon l'invention doivent répondre à des caractéristiques physiques précises qui sont fonction principalement du principe actif et de son dosage. Les caractéristiques générales essentielles des "FOSI" sont relatives à leur aspect, leur forme, leur dureté, leur poids, leur temps de désagrégation et de dispersion et la température de stockage.

L'aspect des "FOSI" doit être homogène en surface et en profondeur et leur surface doit être unie et lisse.

Les formes des "FOSI" peuvent être extrêmement variées mais leur géométrie doit être compatible avec un démoulage facile et avec la voie d'administration.

La consistance des "FOSI" peut être variable. Généralement, elle doit être telle que la "FOSI" résiste aux manipulations de l'utilisateur ou aux chocs par exemple, résistance à une chute de 1 mètre du sol. Par ailleurs, leur cohésion doit être telle qu'elles peuvent être retirées de leur contenant sans nuire à leur intégrité physique.

Le poids des "FOSI" peut être compris entre quelques dizaines de milligrammes et plusieurs centaines de milligrammes, la limite inférieur étant fonction de la précision de la mesure de la quantité de "FOSI" dans le contenant et la limite supérieur étant liée à la taille maximale de la "FOSI" pouvant être absorbée par le patient.

Le temps de désagrégation et de dispersion de la "FOSI" est dans tous les cas inférieur à 45 minutes dans l'eau ou un liquide physiologique artificiel à 37±2°C agitée périodiquement.

La "FOSI" doit rester sous forme solide pendant le stockage c'est-à-dire généralement à une température inférieure à 30°C. Cependant la température de fusion de la composition qui est fonction de la stabilité du principe actif peut être nettement plus élevée.

Selon l'invention, il existe un procédé général de préparation des "FOSI" qui doit cependant être adapté à chaque cas particulier compte tenu des propriétés spécifiques des principes actifs mis en oeuvre.

Le procédé général de préparation des "FOSI" peut se résumer de la manière suivante:

1— à partir d'excipients primaires, c'est-à-dire de substances utilisées habituellement en pharmacie galénique et inscrites dans les pharmacopées, sélectionnés en fonction de leur aptitude à solubiliser ou disperser les principes actifs concernés et choisis parmi l'alcool cétylique, l'acide linoléique, le myristate d'isopropyle, le monostéarate de glycérol, la gélatine, la lécithine, la leucine, la lysine, l'acide glycérophosphorique, le propylèneglycol, le glycérol, l'eau, l'alcool et les terpènes tels que le cinéol, le menthol, l'eucalyptol ou le camphre, est préparé un excipient secondaire ($ES_1^o$) capable de constituer une phase à caractère lipophile, hydrophile ou amphotère selon la nature et les propriétés des principes actifs,

2— le principe actif est solubilisé ou dispersé dans l'excipient secondaire $ES_1^o$ éventuellement en présence d'un excipient primaire favorisant sa solubilisation, pour obtenir une phase φ 1 qui est maintenue liquide à une température suffisante,

3— à partir d'excipients primaires est préparé un excipient secondaire ($ES_2^o$) à caractère hydrophile, lipophile ou amphotère selon la nature de l'excipient secondaire $ES_1^o$ qui constitue une phase φ 2 qui est maintenue liquide à une température suffisante, étant entendu que lorsque $ES_1^o$ est liphophile ou amphotère, lorsque $ES_1^o$ est hydrophile, $ES_2^o$ est lipophile ou amphotère et lorsque $ES_1^o$ est amphotère $ES_2^o$ est lipophile, hydrophile ou amphotère,

4— la phase φ 1 (ou φ 2) est alors dispersée dans la phase φ 2 (ou φ 1) à la même température pour donner un mélange φ 3 de consistance homogène liquide à une température supérieure à la température ambiante de stockage,

5— après refroidissement de la phase φ 3 à une température voisine de la température de solidification, celle-ci est coulée dans des récipients appropriés pour donner des "FOSI" unitaires qui se solidifient par refroidissement en prenant la cohésion requise pour leur manipulation ultérieure, et

6— le récipient contenant les "FOSI" est fermé selon tout moyen approprié, par exemple par thermoformage ou extrusion.

Généralement, l'intégralité du procédé est mis en oeuvre sans précautions particulières quant à la température ambiante et à l'humidité relative.

Il est important de signaler que les excipients convenant pour la fabrication des "FOSI" ne sont pas utilisables pour la fabrication des suppositoires ou des "LYOCS". Inversement, les excipients utilisés pour ces types d'administration tels que les triglycérides, les polyéthylèneglycols, ou le lactose ne conviennent pas pour la fabrication des "FOSI".

Il est particulièrement intéressant de choisir les proportions des différents excipients de façon à obtenir des mélanges eutectiques, c'est-à-dire des mélanges d'excipients capables de donner des systèmes fondus ou liquéfiés à des températures plus basses que les températures de fusion de chacun des constituants dans le but d'éviter d'éventuelles dégradations du ou des principes actifs.

Pour la mise en oeuvre du procédé selon l'invention, il peut être intéressant de préparer des excipients secondaires de référence qui pourront être modifiés en fonction des propriétés spécifiques des principes actifs à formuler. Compte tenu des caractéristiques connues d'un produit actif, il sera possible de choisir le type d'excipient secondaire qui permettra la formulation la plus appropriée.

Les propriétés physico-chimiques essentielles, qui sont prises en considération pour le choix de l'excipient secondaire, sont la solubilité, le point de fusion, le caractère lipophile, hydrophile ou amphotère et la stabilité en fonction de la température, mais d'autres propriétés, telles que la forme cristalline, peuvent intervenir. L'excipient secondaire $ES_1^o$ sera donc choisi de telle manière que la dissolution du principe actif soit réalisée à une température aussi basse que possible. Le principe actif en solution dans l'excipient secondaire $ES_1^o$ constitue la phase φ 1. Dans la grande majorité des cas, la phase φ 1 ainsi obtenue ne peut pas être coulée directement sous forme de "FOSI" car, soit, après solidification, sa solubilité dans l'eau ou les liquides physiologiques artificiels est pratiquement nulle ou trop faible, soit elle constitue une phase liquide non solidifiable à température ambiante. Il est donc nécessaire de compléter la phase φ 1 par une phase φ 2, constituée d'un excipient secondaires $ES_2^o$, qui constitue un support capable de maintenir la phase φ 1 à l'état liquide à une température généralement supérieure à 35°C, de conduire à la solidification à la température ambiante de façon à former la "FOSI" qui sera soluble dans l'eau ou les liquides physiologiques naturels ou artificiels.

Par ailleurs, il peut être nécessaire d'ajouter à la phase φ 1 ou φ 2 des excipients primaires capables de fixer les éléments volatils tels que certains terpènes.

Selon la présente invention, la présentation définitive du médicament est obtenue par dépôt, de manière connue, d'une feuille souple et déchirable sur les récipients contenant les "FOSI" unitaires. La feuille souple et déchirable doit être compatible avec la "FOSI". Elle peut être constituée d'une feuille mince d'aluminium ou de ses alliages. De manière préférée, la nouvelle forme pharmaceutique est conditionnée dans des plaquettes alvéolées.

Les "FOSI" selon la présente invention sont particulièrement utiles pour la préparation de doses unitaires contenant une dose faible de produit actif (corticoïdes, antihistaminiques, vitamines liposolubles, ...) ou un produit fragile tel qu'un aminoacide (méthionine, cystéine, cystine), une protéine ou une enzyme (pancréatine, trypsine, ...).

Les "FOSI" selon la présente invention peuvent être utilisés, par le choix judicieux des excipients, pour la formulation de principes actifs dont la libération dans l'organisme doit être contrôlée et progressive dans le temps.

Les exemples suivants, donnés à titre non limitatif, montrent comment l'invention peut être mise en pratique. Les proportions des divers constituants sont exprimées en poids.

A—Préparation de "FOSI" neutres c'est-à-dire sans produit actif

Exemple 1—Préparation d'une "FOSI" neutre à caractère lipophile

On prépare sous agitation lente un mélange de 3 parties d'eau, 6 parties d'éthanol, 5 parties de propylèneglycol et 5 parties de lécithine à une température voisine de 20°C pour obtenir une phase φ 2.

A 70 parties d'alcool cétylique fondu à 50°C, on ajoute 5 parties d'acide linoléique et 2 parties de myristate d'isopropyle. A la solution ainsi obtenue on ajoute une solution obtenue en dissolvant 3 parties de cinéol, dans l'ordre, 0,25 partie de menthol, 0,5 partie d'eucalyptol et 0,25 partie de camphre à une température voisine de 20°C. On obtient ainsi une phase φ 1.

La phase φ 1 est ajoutée à la phase φ 2 sous agitation lente à une température comprise entre 45 et 50°C.

On obtient ainsi une solution homogène d'un excipient ayant un caractère lipophile.

Exemple 2—Préparation d'une "FOSI" neutre à caractère lipophile

On obtient une phase φ 2 ar fusion de 80 parties de monostéarate de glycérol.

On prépare un mélange constitué de 1 partie de lécithine, 1 partie d'acide glycérophosphorique et 1 partie de propylèneglycol à une température comprise entre 45 et 50°C. A cette solution on ajoute, à une température comprise entre 50 et 55°C une solution obtenue en dissolvant dans 7 parties d'alcool à 95°, 5 parties de cinéol et 5 parties d'eucalyptol. On obtient ainsi une phase φ 1 qui est ajoutée sous agitation lente à une température comprise entre 50 et 55°C à la phase φ 2. On obtient ainsi une solution homogène d'un excipient ayant un caractère lipophile.

Exemple 3—Préparation d'une "FOSI" neutre à caractère lipophile

On prépare sous agitation lente à une température de 20°C un mélange de 1 partie d'alcool à 95°, 1 partie de cinéol, 1 partie de menthol, 1 partie d'eucalyptol et 1 partie de camphre auquel on ajoute 7 parties de lécithine chauffée à une température comprise entre 35 et 40°C pour obtenir une phase φ 1.

On prépare sous agitation lente à la même température un mélange de 85 parties de myristate d'isopropyle, 1 partie d'alcool cétylique, 1 partie d'acide linoléique et 1 partie de monostéarate de glycérol pour obtenir une phase φ 2.

La phase φ 1 est ajoutée sous agitation à la même température (40°C) à la phase φ 2. On obtient ainsi une solution homogène d'un excipient ayant un caractère lipophile.

Exemple 4—Préparation d'une "FOSI" neutre à caractère hydrophile

On prépare sous agitation lente, à une température de 50°C, un mélange de 60 parties d'eau, 5 parties de gélatine, 1 partie de lécithine, 5 parties de propylèneglycol pour obtenir une phase φ 1.

On prépare sous agitation lente, à la même température, un mélange de 10 parties d'alcool à 95°, 3 parties de cinéol, 0,5 partie de menthol, 5 parties d'eucalyptol, 0,5 partie de camphre et de 1 partie de myristate d'isopropyle pour obtenir une phase φ 2.

On ajoute la phase φ 2 à la phase φ 1 sous agitation à la même température (50°C). On obtient ainsi une solution homogène d'un excipient ayant un caractère hydrophile.

Exemple 5—Préparation d'une "FOSI" neutre à caractère hydrophile

On prépare sous agitation lente, à une température de 50°C, un mélange de 75,5 parties d'eau, 5 parties de gélatine, 2 parties de lécithine, 0,5 partie de lysine, 0,5 partie de leucine et 5 parties de propylèneglycol pour obtenir une phase φ 1.

On prépare sous agitation lente, à la même température (50°C), un mélange de 5 parties d'alcool à 95°, 5 parties de cinéol, 0,5 partie de menthol, 0,5 partie d'eucalyptol et 0,5 partie de camphre pour obtenir une phase φ 2.

La phase φ 2 est ajoutée sous agitation, à la même température, à la phase φ 1. On obtient ainsi une solution homogène ayant un caractère hydrophile.

Exemple 6—Préparation d'une "FOSI" neutre à caractère amphotère

On fond à une température comprise entre 55 et 60°C, 1 partie de monostéarate de glycérol, et on ajoute sous agitation lente à 55—60°C un mélange de 20 parties d'alcool acétylique et 1 partie de myristate d'isopropyle pour obtenir une phase φ 2.

On prépare sous agitation lente, à une température de 45 à 50°C, un mélange de 20 parties d'eau, 1 partie de gélatine, 5 parties de lécithine, 1 partie de lysine, 1 partie de leucine, 10,5 parties de propanetriol et 10,5 parties de propylèneglycol, auquel on ajoute un mélange, à la même température, de 25 parties d'alcool à 95°, 3 parties de cinéol, 0,25 partie de menthol, 0,5 partie d'eucalyptol et 0,25 partie de camphre pour obtenir une phase φ 1.

Les phases φ 1 et φ 2 sont chauffées à une température comprise entre 40 et 50°C, puis la phase φ 1 est ajoutée à la phase φ 2 à la même température sous agitation. On obtient ainsi une solution homogène ayant un caractère amphotère.

B—Préparation de "FOSI" contenant un produit actif

Exemple 7—

On prépare des "FOSI" contenant 150 mg de kétoprofène pour un poids total de 1000 mg.

On prépare sous agitation lente, à une température comprise entre 60 et 65°C, un mélange de 2 parties d'alcool à 95°, 10 parties de propylèneglycol, 0,5 partie de menthol et 15 parties d'eucalyptol auquel on ajoute 15 parties de kétoprofène et 0,1 partie de monostéarate de glycérol. On obtient ainsi une phase φ 1.

On prépare, à la même température, un mélange de 37,4 parties d'eau, 18 parties de gélatine et 2 parties de lécithine. On obtient une phase φ 2. La phase φ 1 est ajoutée à la phase φ 2 à la même température de façon à obtenir un mélange homogène.

Ce mélange est coulé sur une plaque alvéolée de telle manière que chaque alvéole contienne 1000 mg du mélange. Après refroidissement on obtient des "FOSI" rigides, blanchâtres ayant une saveur faible terpénique et une odeur caractéristique des terpènes volatils utilisés.

Une "FOSI" unitaire ainsi obtenue a un temps de désagrégation èt de dispersion dans l'eau à 37±2°C inférieur à 20 minutes.

Exemple 8—

On prépare des "FOSI" contenant 100 mg de kétoprofène pour un poids total de 1000 mg.

On prépare, sous agitation lente, à 20°C environ, un mélange de 2 parties d'alcool à 95° et 10 parties de propylèneglycol que l'on chauffe à une température comprise entre 60 et 65°C et auquel on ajoute un mélange de 15 parties d'eucalyptol, 0,5 partie de menthol et 5 parties de propanetriol. Au mélange ainsi obtenu, à une température comprise entre 60 et 65°C, on ajoute un mélange de 10 parties de kétoprofène et 0,2 partie de monostéarate de glycérol. On obtient ainsi une phase φ 1.

On prépare, à une température comprise entre 60 et 65°C, un mélange de 37,3 parties d'eau, 18 parties de gélatine et 2 parties de lécithine. On obtient ainsi une phase φ 2.

On ajoute la phase φ 1 à la phase φ 2 à une température comprise entre 60 et 65°C de façon à obtenir un mélange homogène.

Ce mélange est coulé sur une plaque alvéolée de telle manière que chaque alvéole contienne 1000 mg du mélange. Après refroidissement, on obtient des "FOSI" blanchâtres de consistance molle, ayant une saveur faible terpénique et une odeur caractéristique des terpènes volatils utilisés.

Une "FOSI" unitaire ainsi obtenue a un temps de désagrégation et de dispersion inférieur à 5 minutes dans l'eau à une température de 37±2°C.

Exemple 9—

On prépare des "FOSI" contenant 150 mg de kétoprofène pour un poids total de 1000 mg.

On prépare sous agitation lente, à une température de 20°C environ, un mélange de 20 parties d'eucalyptol, 0,5 partie de menthol, 0,5 partie de camphre et 2 parties d'alcool pour obtenir un excipient secondaire ES, qui est chauffé à une température comprise entre 50 et 55°C auquel on ajoute 15 parties de kétoprofène. On obtient ainsi une phase liquide homogène φ 1.

On prépare, à température comprise entre 40 et 50°C, un mélange de 36 parties d'eau, 10 parties de gélatine, 5 parties de lécithine, 0,5 partie de monostéarate de glycérol, 5 parties de propanetriol et 5 parties de propylèneglycol. On obtient ainsi une phase homogène φ 2 que l'on ajoute, à la même température de 50°C, à la phase φ 1.

Ce mélange est coulé sur une plaque alvéolée de telle manière que chaque alvéole contienne 1000 mg du mélange. Après refroidissement, on obtient das "FOSI" blanchâtres, de consistance intermédiaire ayant une saveur faible terpénique et une odeur caractéristique des terpènes volatils utilisés.

Une "FOSI" unitaire ainsi obtenue a un temps de désagrégation et de dispersion inférieur à 10 minutes dans l'eau à une température de 37±2°C.

Exemple 10—

On prépare des "FOSI" contenant 2,5 mg de prednisone pour un poids total de 100 mg.

On prépare sous agitation lente, à une température de 30°C, un mélange de 10 parties d'eucalyptol, 10 parties de propanetriol et 1 partie de "Tween 80"®, auquel on ajoute 2,5 parties de prednisone pour obtenir une phase φ 1 qui est chauffée à 45°C.

On prépare, à la même température, un mélange de 63,5 parties d'eau, 2 parties de gélatine et 10 parties de propanetriol. On obtient ainsi une phase φ 2 homogène qui est ajoutée, à la même température de 45°C, à la phase φ 1 pour obtenir un mélange liquide homogène.

Ce mélange est coulé sur une plaque alvéolée de telle manière que chaque alvéole contienne 100 mg du mélange. Après refroidissement, on obtient des "FOSI" blanchâtres, de consistance molle, ayant une saveur faible terpénique et une odeur caractéristique des terpènes volatils utilisés.

Une "FOSI" unitaire a un temps de désagrégation et de dispersion inférieur à 10 minutes dans l'eau à 37±2°C.

Exemple 11—

En opérant comme dans l'exemple 10 mais en remplaçant la prednisone par la prednisolone, on obtient des "FOSI" contenant 2,5 mg de prednisolone pour un poids total de 100 mg.

**Revendication pour les Etats Contractants: BE, CH, DE, FR, GB, IT, LI, LU, NL, SE**

Forme galénique solide pour administration orale constituée d'un ou plusieurs principes actifs dissous ou dispersés dans un mélange d'excipients, liquéfiable à une température supérieure à 35°C, solide à température ambiante et pouvant libérer le ou les principes actifs après dissolution dans l'eau ou un liquide physiologique caractérisée en ce que, les excipients étant choisis parmi l'alcool cétylique, l'acide linoléique, le myristate d'isopropyle, le monostéarate de glycérol, la gélatine, la lécithine, la leucine, la lysine, l'acide glycérophosphrique, le propylèneglycol, le glycérol, l'eau, l'éthanol et les terpènes tels que le cinéol, le menthol, l'eucalyptol et le camphre, elle est obtenue en réalisant la succession des opérations suivantes:

a) on solubilise le ou les principes actifs dans un excipient ou un mélange d'excipients à caractère lipophile, hydrophile ou amphotère selon la nature et les propriétés du ou des principes actifs, maintenu liquide à une température supérieure à 35°C,

b) on mélange la phase liquide ainsi obtenue à un excipient ou un mélange d'excipients lipophile, hydrophile ou amphotère étant entendu que, lorsque le ou les principes actifs sont dissous dans un excipient ou un mélange d'excipients lipophiles, le second excipient est hydrophile ou amphotère, lorsque le ou les principes actifs sont dissous dans un excipient ou un mélange d'excipients hydrophiles, le second excipient est lipophile ou amphotère et lorsque le ou les principes actifs sont dissous dans une excipient ou un mélange d'excipients amphotère, le second excipient est lipophile, hydrophile ou amphotère, de façon que la solution obtenue soit liquide à une température supérieure à 35°C,

c) coule le liquide ainsi obtenu dans un contenant pouvant permettre la réalisation de plusieurs doses unitaires

d) laisse refroidir jusqu'à solidification la nouvelle forme galénique et,

e) ferme le contenant par tout dispositif approprié tel que le thermoformage ou l'extrusion.

**Revendication pour l'Etat Contractant: AT**

Procédé de préparation d'une forme galénique solide pour administration orale constituée d'un ou plusieurs principes actifs dissous ou dispersés dans un mélange d'excipients liquéfiable à une température supérieure à 35°C, solide à température ambiante, compatible avec le ou les principes actifs et pouvant libérer le ou les principes actifs après dissolution dans l'eau ou un liquide physiologique, caractérisé en ce que, les excipients étant choisis parmi l'alcool cétylique, l'acide linoléique, le myristate d'isopropyle, le monostéarate de glycérol, la gélatine, la lécithine, la leucine, la lysine, l'acide glycérophosphorique, le propylèneglycol, le glycérol, l'eau, l'éthanol et les terpènes tels que le cinéol, le menthol, l'eucalyptol et le camphre,

a) on solubilise le ou les principes actifs dans un excipient ou un mélange d'excipient à caractère lipophile, hydrophile ou amphotère selon la nature et les propriétés du ou des principes actifs, maintenu liquide à une température supérieure à 35°C,

b) on mélange la phase liquide ainsi obtenue à un excipient ou un mélange d'excipients lipophile, hydrophile ou amphotère étant entendu que, lorsque le ou les principes actifs sont dissous dans un excipient ou un mélange d'excipients lipophile, le second excipient est hydrophile ou amphotère, lorsque le ou les principes actifs sont dissous dans un excipient ou un mélange d'excipients hydrophile, le second excipient est lipophile ou amphotère, et lorsque le ou les principes actifs sont dissous dans un excipient ou un mélange d'excipients amphotère, le second excipient est lipophile, hydrophile ou amphotère, de façon que la solution obtenue soit liquide à une température supérieure à 35°C,

c) coule le liquide ainsi obtenu dans un contenant pourvant permettre la réalisation de plusieurs doses unitaires,

d) laisse refroidir jusqu'à solidification la nouvelle forme galénique et,

e) ferme le contenant par tout dispositif approprié tel que le thermoformage ou l'extrusion.

**Patentanspruch für die Vertragsstaaten: BE, CH, DE, FR, GB, IT, LI, LU, NL, SE**

Feste galenische Form zur oralen Verabreichung, bestehend aus einem oder mehreren Wirkstoffen, die gelöst oder dispergiert sind in einem Gemisch von Grundmassen (Exzipienten), das bei einer Temperatur oberhalb 35°C verflüssigbar und bei Umgebungstemperatur fest ist und den oder die Wirkstoffe nach Auflösung in Wasser oder einer physiologischen Flüssigkeit freisetzen kann, dadurch gekennzeichnet, daß die Exzipienten ausgewählt sind unter Cetylalkohol, Linolsäure, Isopropylmyristat, Glycerinmonostearat, Gelatine, Lecithin, Leucin, Lysin, Glycerophosphorsäure, Propylenglycol, Glycerin, Wasser, Ethanol und den Terpenen wie Cineol, Menthol, Eucalyptol und Campher und sie erhalten wird, indem die folgenden Arbeitsgänge nacheinander durchgeführt werden:

a) Man macht das oder die Wirkstoffe in einem Exzipienten oder einem Gemisch von Exzipienten mit lipophilem, hydrophilem oder amphoterem Charakter, je nach der Natur und den Eigenschaften des oder der Wirkstoffe, der bei einer Temperatur oberhalb 35°C flüssig gehalten wird, löslich,

b) man vermischt die so erhaltene flüssige Phase mit einem lipophilen, hydrophilen oder amphoteren Exzipienten oder einem Gemisch von Exzipienten, wobei, falls der oder die Wirkstoffe in einem lipophilem Exzipienten oder einem Gemisch lipophiler Exzipienten gelöst ist, der zweite Exzipient hydrophil oder amphoter ist, falls der oder die Wirkstoffe in einem hydrophilen Exzipienten oder einem Gemisch hydrophiler Exzipienten gelöst ist, der zweite Exzipient lipophil oder amphoter ist, und, falls der oder die Wirkstoffe in einem amphoteren Exzipienten oder einem Gemisch amphoterer Exzipienten gelöst sind, der zweite Exzipient lipophil, hydrophil oder amphoter ist, derart, daß die erhaltene Lösung bei einer Temperatur oberhalb von 35°C flüssig ist,

c) man gießt die so erhaltene Flüssigkeit in ein Behältnis, das die Realisierung von mehreren Einheitsdosen ermöglichen kann,

d) läßt die neue galenische Form bis zur Verfestigung abkühlen und

e) schließt das Behältnis durch jede geeignete Vorrichtung wie Thermoverformung oder Extrudieren.

**Patentanspruch für den Vertragsstaat: AT**

Verfahren zur Herstellung einer festen galenischen Form zur oralen Verabreichung, bestehend aus einem oder mehreren aktiven Bestandteil(en), der (die) in einem oder mehreren bei einer Temperatur oberhalb Umgebungstemperatur, z.B. 35°C, verflüssigbaren Bindemittel(n), das (die) mit dem oder den aktiven Bestandteil(en) verträglich und bei Umgebungstemperatur fest ist (sind) und den oder die aktiven Bestandteil(e) nach Auflösung in Wasser oder einer physiologischen Flüssigkeit freizusetzen vermag (vermögen), gelöst oder dispergiert ist (sind), dadurch gekennzeichnet, daß man die Bindemittel auswählt aus Cetylalkohol, Linolsäure, Isopropylmyristat, Glycerinmonostearat, Gelatine, Lecithin, Leucin, Lysin, Glycerophosphorsäure, Propylenglykol, Glycerin, Wasser, Alkohol und den Terpenen wie Cineol, Menthol, Eucalyptol oder Kampfer und

a) den aktiven Bestandteil in einem bei einer Temperatur von mehr als 35°C flüssig gehaltenen Bindemittel oder Bindemittelgemisch mit lipophilem, hydrophilem oder amphoterem Charakter, je nach der Art und den Eigenschaften des aktiven Bestandteils oder der aktiven Bestandteile löslich macht,

b) die so erhaltene flüssige Phase mit einem lipophilen, hydrophilen oder amphoteren Bindemittel oder einer Bindemittelmischung mischt, wobei selbstverständlich, wenn der aktive Bestandteil oder die aktiven Bestandteile in einem lipophilen Bindemittel oder Bindemittelgemisch gelöst sind, das zweite Bindemittel hydrophil oder amphoter ist, wenn der aktive Bestandteil oder die aktiven Bestandteile in einem hydrophilen Bindemittel oder Bindemittelgemisch gelöst sind, das zweite Bindemittel lipophil oder amphoter ist, und wenn der aktive Bestandteil oder die aktiven Bestandteile in einem amphoteren Bindemittel oder Bindemittelgemisch gelöst sind, der zweite Bestandteil lipophil, hydrophil oder amphoter ist, derart, daß die erhaltene Lösung bei einer Temperatur oberhalb 35°C flüssig ist,

c) die so erhaltene Flüssigkeit in einen Behälter gießt, der die Herstellung mehrerer Einheitsdosen zuläßt,

d) die neue galenische Form bis zur Erstarrung abkühlen läßt und

e) den Behälter mittels irgendeiner geeigneten Einrichtung, z.B. durch Wärmeformen oder Strangpressen, schließt.

**Claim for the Contracting States: BE, CH, DE, FR, GB, IT, LI, LU, NL, SE**

Solid galenical form for oral administration consisting of one or more active principles dissolved or dispersed in a mixture of excipients, which can be liquefied at a temperature above 35°C, is solid at room temperature and can release the active principle or principles after dissolution in water or a physiological liquid, characterised in that, with the excipients being chosen from cetyl alcohol, linoleic acid, isopropyl myristate, glycerol monostearate, gelatin, lecithin, leucine, lysine, glycerophosphoric acid, propylene glycol, glycerol, water, ethanol and terpenes such as cineole, menthol, eucalyptole and camphor, the galenical form is obtained by carrying out in succession the following operations:

a) the active principle or principles are solubilised in an excipient or mixture of excipients having lipophilic, hydrophilic or amphoteric character, depending on the nature and properties of the active principle or principles, the mixture being maintained liquid at a temperature above 35°C,

b) the liquid phase thereby obtained is mixed with a lipophilic, hydrophilic or amphoteric excipient or mixture of excipients, with the proviso that, when the active principle or principles is/are dissolved in an excipient or mixture of excipients which are lipophilic, the second excipient is hydrophilic or amphoteric, when the active principle or principles is/are dissolved in an excipient or mixture of excipients which are hydrophilic, the second excipient is lipophilic or amphoteric, and when the active principle or principles are dissolved in an amphoteric excipient or mixture of excipients, the second excipient is lipophilic, hydrophilic or amphoteric, so that the solution obtained is liquid at a temperature above 35°C,

c) the liquid thereby obtained is poured into a recipient which can enable several unit doses to be produced,

d) the new galenical form is allowed to cool until it solidifies and,

e) the recipient is closed by means of any suitable arrangement, such as thermoforming or extrusion.

**Claim for the Contracting State: AT**

Process for preparing a solid galenical form for oral administration consisting of one or more active principles dissolved or dispersed in a mixture of excipients, which can be liquefied at a temperature above 35°C, is solid at room temperature, is compatible with the active principle or principles and can release the active principle or principles after dissolution in water or a physiological liquid, characterised in that, with the excipients being chosen from cetyl alcohol, linoleic acid, isopropyl myristate, glycerol monostearate, gelatin, lecithin, leucine, lysine, glycerophosphoric acid, propylene glycol, glycerol, water, ethanol and terpenes such as cineole, menthol, eucalyptole and camphor,

a) the active principle or principles are solubilised in an excipient or excipient mixture having lipophilic, hydrophilic or amphoteric character, depending on the nature and properties of the active principle or principles, the mixture being maintained liquid at a temperature above 35°C,

b) the liquid phase thereby obtained is mixed with a lipophilic, hydrophilic or amphoteric mixture of excipients, with the proviso that, when the active principle or principles is/are dissolved in a lipophilic excipient or mixture of excipients, the second excipient is hydrophilic or amphoteric, when the active principle or principles is/are dissolved in a hydrophilic excipient or mixture of excipients, the second excipient is lipophilic or amphoteric, and when the active principle or principles is/are dissolved in an amphoteric excipient or mixture of excipients, the second excipient is lipophilic, hydrophilic or amphoteric, so that the solution obtained is liquid at a temperature above 35°C,

c) the liquid thereby obtained is poured into a recipient which can enable several unit doses to be produced,

d) the new galenical form is allowed to cool until it solidifies and,

e) the recipient is closed by means of any suitable arrangement, such as thermoforming or extrusion.